# EUROPEAN PATENT APPLICATION

(11) **EP 4 458 815 A1**
(43) Date of publication of application: **06.11.2024**
(21) Application number: 22913672.6
(22) Date of filing: 17.10.2022
(51) Int. Cl.: C07D 277/06, A61K 31/426, A61P 7/00, A61P 7/06

(54) **CRYSTALLINE FORM OF LEUCOGEN AND METHOD FOR PREPARATION THEREOF AND USE THEREOF**

(30) Priority: 30.12.2021 CN 202111654237; 30.12.2021 CN 202111654303
(71) Applicant: Jiangsu Jibeier Pharmaceutical Co. Ltd., Zhenjiang, Jiangsu 212009 (CN)
(72) Inventor: WU, Xiugen, Zhenjiang, Jiangsu 212009 (CN); LI, Zhaoguang, Zhenjiang, Jiangsu 212009 (CN); LI, Haidao, Zhenjiang, Jiangsu 212009 (CN); NIE, Liyun, Zhenjiang, Jiangsu 212009 (CN); LIU, Yunfeng, Zhenjiang, Jiangsu 212009 (CN); CAO, Xu, Zhenjiang, Jiangsu 212009 (CN); WEI, Furong, Zhenjiang, Jiangsu 212009 (CN)
(74) Representative: Müller-Boré & Partner Patentanwälte PartG mbB
(86) International application number: PCT/CN2022/125692
(87) International publication number: WO 2023/124408

(57) **Abstract**

The present invention relates to the field of compound crystal forms, and particularly provides a leucogen crystalline form A, crystalline form B, a method for preparation of the crystalline form B, a pharmaceutical composition, and an application. The crystalline form A and the crystalline form B provided by the present invention have the advantages of good stability, high solubility in water, and good bioavailability.

## Description

### TECHNICAL FIELD

The present disclosure pertains to the technical field of crystal preparation in medicinal chemistry, and specifically relates to a crystalline form A and a crystalline form B of leucogen and further to preparation methods of the crystalline form A and the crystalline form B, compositions comprising the same, and use thereof.

### BACKGROUND

Leucogen is a typical thiazole carboxylic acid leukocyte-boosting drug, with the chemical name of 2-(α-phenyl-α-ethoxycarbonyl-methyl)thiazolidine-4-carboxylic acid. It is effective in promoting proliferation of leukocytes, and is widely used for treatment of leukopenia caused by radiotherapy and chemotherapy in the course of treating malignant tumors. Leucogen is a cysteine derivative, which is quickly absorbed after taking, thereby enhancing the function of the body's hematopoietic system. At present, it is used most commonly to prevent and treat leukopenia, aplastic anemia, and thrombocytopenia and the like caused by various reasons.

Thiazolidine 4-carboxylic acid and its derivatives are generally prepared by a method of condensing L-cysteine hydrochloride and the corresponding aldehyde under appropriate conditions. As far as leucogen is concerned, ethyl phenylacetate reacts with ethyl formate in the presence of sodium alkoxide to produce ethyl α-formylphenylacetate; subsequently, ethyl α-formylphenylacetate is cyclized with L-cysteine hydrochloride; the product precipitates at room temperature and is subjected to filtration, washing, and recrystallization to give leucogen. The product is white crystalline powder and is insoluble in water and ethanol.

The patent application numbered CN200410054101.3 discloses preparation of the leucogen raw material from ethyl phenylacetate, ethyl formate, ether, and sodium metal by condensation, acidification, and cyclization, and describes in details a crystal preparation and purification method of controlling the pH at about 7 in the cyclization step, refluxing by heating the reactants in the presence of water and ethanol, then cooling to crystallize, and subjecting to spin filtration, washing the crystal with distilled water and ethanol in sequence and then with a small amount of ether.

The patent application numbered CN200610037819.0 discloses a synthesis process of subjecting ethyl phenylacetate and ethyl formate to condensation under the action of a condensing agent and to acidification to obtain ethyl formylphenylacetate, and cyclizing the ethyl formylphenylacetate and cysteine to produce a leucogen active pharmaceutical ingredient; the preparation and purification method for the leucogen active pharmaceutical ingredient crystal is basically the same as the above method with the difference in solvents for washing the crystal, namely, this crystal is washed with ether, ethanol, and water.

The patent application numbered CN201110357853.7 discloses a method of subjecting ethyl phenylacetate and ethyl formate to a condensation reaction in the presence of particular solvent and condensing agent, acidifying the reaction solution after extraction and purification, and then crystallizing at a low temperature to afford a solid intermediate ethyl α-formylphenylacetate with a high purity; subsequently, reacting the ethyl α-formylphenylacetate with L-cysteine hydrochloride in the presence of water and acetone, subjecting the resultant to cooling upon completion of the reaction, suction filtration, repeated washing with water and acetone, and vacuum drying to afford the product.

Furthermore, Fu Lin *et al.* have reported the method for washing products with water and ethanol, respectively. Wang Yanxia *et al.* have reported the method for preparing white crystalline leucogen by reacting in the presence of water and ethanol to afford a product, filtering the product, washing the filter cake with water and petroleum ether respectively, and then drying, where the melting points of the products are 158.1°C to 158.7°C. Lu Qiang and Wang Yanyan have reported the method of refluxing by heating the reaction solution containing water and ethanol and then cooling to 15°C to 18°C, adding water and stirring for 6 h until the product fully precipitates, then filtering the product, and washing with ethanol, water (pH = 3.0), and ethanol. Zhao Ling and Yang Bo have reported the method of using water and ethanol as solvents to prepare a reaction product, cooling the reaction product to crystalize, filtering the crystal after washing with ether, ethanol and water, and drying to afford the product.

In spite of possessing many advantages in prevention and treatment of hematological system diseases such as leukopenia, thrombopenia, and aplastic anemia caused by various reasons, leucogen is still faced with some problems in practical application:
1) the available crystalline forms of leucogen have a special odor, which has a certain impact on the patients' medication compliance;
2) the commonly used crystalline forms of leucogen have a small bulk density, which affects the drug release to some extent; and
3) leucogen contains methyl ester impurities, which will affect the drug potency.

The above deficiencies restrict further clinical promotion and use of leucogen. Therefore, how to improve the above properties of leucogen simply and effectively is an extremely critical factor for its further development and application. Currently, the commonly used methods for improving the properties of leucogen often require a change in its structure or addition of additives, etc., which are not only costly, but also have uncertain effects. As such, it is urgent to solve the above problems in the process of drug research and development for leucogen.

### SUMMARY

An objective of the present disclosure is to overcome the problems existing in the prior art, for example, leucogen contains methyl ester impurities and has special odor, poor water solubility, etc. The present disclosure provides the technical solutions of preparing a crystalline form A using water and *N*,*N*-dimethylformamide as solvents, and preparing a crystalline form B using *N*,*N*-dimethylformamide as a solvent. The crystalline form A and crystalline form B have the advantages of a high water solubility value and a high dissolution rate, and have excellent bioactivity, which are suitable for application in the production process of pharmaceutical preparations. Drugs having the same chemical structure afford different crystals due to different crystallization conditions, and different crystals often have different physical and chemical properties, which provide more selectivity for the development of preparations.

According to the first aspect of the present disclosure, provided is the following technical solution: a crystalline form A of leucogen, wherein an X-ray powder diffraction pattern of the crystalline form A shows at least three diffraction peaks at the following 2θ angles: 6.215°±0.2°, 9.738°±0.2°, 13.130°±0.2°, 18.072°±0.2°, 19.516°±0.2°, 21.446°±0.2°, 22.331°±0.2°, 23.688°±0.2°, and 29.390°±0.2°.

Further, the X-ray powder diffraction pattern of the crystalline form A shows at least four diffraction peaks at the following 2θ angles: 6.215°±0.2°, 9.738°±0.2°, 13.130°±0.2°, 18.072°±0.2°, 19.516°±0.2°, 21.446°±0.2°, 22.331°±0.2°, 23.688°±0.2°, and 29.390°±0.2°.

Further, the X-ray powder diffraction pattern of the crystalline form A shows at least five diffraction peaks at the following 2Θ angles: 4.839°±0.2°, 6.215°±0.2°, 9.738°±0.2°, 13.130°±0.2°, 18.072°±0.2°, 18.625°±0.2°, 19.516°±0.2°, 21.446°±0.2°, 22.331°±0.2°, 23.688°±0.2°, 25.020°±0.2°, 26.341°±0.2°, 27.079°±0.2°, 28.088°±0.2°, 29.390°±0.2°, 31.200°±0.2°, and 37.996°±0.2°.

Further, the X-ray powder diffraction pattern of the crystalline form A shows at least seven diffraction peaks at the following 2Θ angles: 4.839°±0.2°, 6.215°±0.2°, 9.738°±0.2°, 13.130°±0.2°, 18.072°±0.2°, 18.625°±0.2°, 19.516°±0.2°, 21.446°±0.2°, 22.331°±0.2°, 23.688°±0.2°, 25.020°±0.2°, 26.341°±0.2°, 27.079°±0.2°, 28.088°±0.2°, 29.390°±0.2°, 31.200°±0.2°, and 37.996°±0.2°.

Further, the melting point is 161°C to 162°C.

The present disclosure further provides a method for preparing the crystalline form A of leucogen as described above, wherein a leucogen compound (active pharmaceutical ingredient, API) is dissolved with stirring in the presence of *N*,*N*-dimethylformamide, and subjected to cooling, water precipitation, suction filtration, and washing to afford a filter cake, and the filter cake is dried to afford the crystalline form A.

Further, temperatures for the dissolution and the stirring are both not higher than 15°C. Even further, the temperatures for the dissolution and the stirring are 8°C to 12°C.

Further, the mass/volume ratio of the leucogen compound to the *N*,*N*-dimethylformamide is 1g: (5-25)mL, preferably 1g: (10-15)mL.

Further, the volume ratio of the water to the *N*,*N-*dimethylformamide is 1:1.

Further, the drying is air-blowing drying at 60°C to 80°C.

Even further, the drying is air-blowing drying at 70°C.

The present disclosure further provides a pharmaceutical composition, comprising an effective amount of the crystalline form A of leucogen as described above.

Even further, the pharmaceutical composition further comprises a pharmaceutically acceptable excipient.

The present disclosure further provides use of the crystalline form A of leucogen as described above or the pharmaceutical composition as described above in the preparation of a medicament for preventing and treating a hematological system disease.

Further, the hematological system disease is leukopenia, thrombopenia or aplastic anemia caused by various reasons.

Compared with the prior art, the crystalline form A provided in the first aspect of the present disclosure possesses the following remarkable advantages:
1) the technical solution of the present disclosure uses water and *N*,*N*-dimethylformamide as solvents to prepare the crystalline form A of leucogen under low temperature conditions, which has the advantages of a high water solubility value and a high water dissolution rate, in particular increasing the solubility in water to 240 µg/mL or above, and greatly reduces the content of impurities; it is thus suitable for use in the production process of pharmaceutical preparations, thereby avoiding the inconvenience in preparation and storage of preparations caused by the low water solubility of leucogen in the prior art; and
2) the crystalline form A of leucogen of the present disclosure has better bioactivity and is more suitable for use in pharmaceutical preparations, as compared to conventional crystalline forms of leucogen API.

According to the second aspect of the present disclosure, provided is the following technical solution: a crystalline form B of leucogen, wherein an X-ray powder diffraction pattern of the crystalline form B shows at least three diffraction peaks at the following 2Θ angles: 8.025°±0.2°, 11.115°±0.2°, 20.310°±0.2°, 22.282°±0.2°, 28.546°±0.2°, 33.667°±0.2°, and 35.671°±0.2°.

Further, the X-ray powder diffraction pattern of the crystalline form B shows at least four diffraction peaks at the following 2θ angles: 8.025°±0.2°, 11.115°±0.2°, 20.310°±0.2°, 22.282°±0.2°, 28.546°±0.2°, 33.667°±0.2°, and 35.671°±0.2°.

Further, the X-ray powder diffraction pattern of the crystalline form B shows at least five diffraction peaks at the following 2Θ angles: 8.025°±0.2°, 11.115°±0.2°, 17.662°±0.2°, 20.310°±0.2°, 22.282°±0.2°, 24.115°±0.2°, 28.546°±0.2°, 33.667°±0.2°, and 35.671°±0.2°.

Further, the X-ray powder diffraction pattern of the crystalline form B shows at least seven diffraction peaks at the following 2θ angles: 8.025°±0.2°, 11.115°±0.2°, 17.662°±0.2°, 20.310°±0.2°, 22.282°±0.2°, 24.115°±0.2°, 28.546°±0.2°, 33.667°±0.2°, and 35.671°±0.2°.

Further, the melting point is 159°C to 160°C.

The present disclosure further provides a method for preparing the crystalline form B of leucogen as described above, wherein a leucogen compound (active pharmaceutical ingredient, API) is heated and dissolved with stirring in the presence of *N*,*N*-dimethylformamide, and cooled to precipitate a solid, the solid is subjected to suction filtration, and a filter cake is washed and dried to afford the crystalline form B.

Further, temperatures for the dissolution and the stirring are both higher than 70°C.

Even further, the temperatures for the dissolution and the stirring are 80°C to 85°C. Further, the cooling is specifically cooling to 10°C to 30°C.

Further, the drying is air-blowing drying at 60°C to 80°C.

Even further, the drying is air-blowing drying at 70°C.

Further, the mass/volume ratio of the leucogen API to the *N*,*N*-dimethylformamide is 1g: (1-10)mL, preferably 1g: (2-5)mL, for example, 1g:2mL.

The present disclosure further provides a pharmaceutical composition, comprising an effective amount of the crystalline form B of leucogen as described above.

Even further, the pharmaceutical composition further comprises a pharmaceutically acceptable excipient.

The present disclosure further provides use of the crystalline form B of leucogen as described above or the pharmaceutical composition as described above in the preparation of a medicament for preventing and treating a hematological system disease.

Further, the hematological system disease is leukopenia, thrombopenia or aplastic anemia caused by various reasons.

Compared with the prior art, the crystalline form B provided in the second aspect of the present disclosure possesses the following remarkable advantages:
1) the technical solution of the present disclosure uses *N*,*N*-dimethylformamide as a solvent to prepare the crystalline form B of leucogen under heating conditions, which has the advantages of a high water solubility value and a high water dissolution rate, in particular increasing the solubility in water to 256 µg/mL or above, and greatly reduces the content of impurities; it is thus suitable for use in the production process of pharmaceutical preparations, thereby avoiding the inconvenience in preparation and storage of preparations caused by the low water solubility of leucogen in the prior art; and
2) the crystalline form B of leucogen of the present disclosure has better bioactivity and is more suitable for use in pharmaceutical preparations, as compared to conventional crystalline forms of leucogen API.

The pharmaceutical composition of the present disclosure may be prepared by combining the compound crystalline form of the present disclosure with a suitable pharmaceutically acceptable excipient, for example, it may be formulated into solid, semi-solid, liquid or gaseous preparations, such as tablets, pills, capsules, powders, granules, ointments, emulsions, suspensions, suppositories, injections, inhalants, gels, microspheres, and aerosols.

Typical routes of administration of the pharmaceutical composition of the present disclosure include, but are not limited to, oral, rectal, topical, inhalation, parenteral, sublingual, intraperitoneal, intramuscular, subcutaneous, and intravenous administration.

The pharmaceutical composition of the present disclosure may be manufactured by a method well-known in the art, such as a conventional mixing, dissolution, granulating, sugar-coating pelletizing, grinding, emulsifying, or freeze-drying method.

In some embodiments, the pharmaceutical composition is in an oral form. For oral administration, the pharmaceutical composition may be formulated by mixing the crystalline form of the active compound with pharmaceutically acceptable excipients. These excipients enable the compounds of the present disclosure to be formulated into tablets, pills, lozenges, sugar coatings, capsules, liquids, gels, slurries, suspensions, etc. for oral administration to patients.

Solid oral compositions may be prepared by a conventional mixing, filling or tablet pressing method. For example, the solid oral composition may be obtained by the following method: mixing the active compound with a solid excipient, and optionally milling the resulting mixture. Suitable excipients include, but are not limited to: binders, diluents, disintegrants, lubricants, glidants, sweeteners or corrigents, etc.

Besides, the present disclosure further provides a method for preventing and/or treating a hematological system disease, comprising administering a therapeutically effective amount of a crystalline form A or crystalline form B of leucogen, or a pharmaceutical composition comprising the same to a mammal, preferably human being, in need thereof. The hematological system disease is leukopenia, thrombopenia or aplastic anemia caused by various reasons.

In the method for administering the crystalline form A or crystalline form B of leucogen, the daily dose of administration is from 0.01 to 100 mg/kg body weight, preferably 0.05 to 50 mg/kg body weight, more preferably 0.1 to 5 mg/kg body weight.

The term "pharmaceutically acceptable excipient" refers to the excipients that do not have a significant irritating effect on organisms and do not impair the biological activity and properties of the active compound. Suitable excipients are well known to a person skilled in the art, e.g., carbohydrates, waxes, water soluble and/or water expandable polymers, hydrophilic or hydrophobic materials, gelatin, oils, solvents, and water.

The "effective amount" or "therapeutically effective amount" means an amount of pharmaceutical active ingredient that, when administered to a person for treatment of a disease, is sufficient to achieve a certain therapeutic effect on the disease.

The term "comprise" and its English variants such as comprises or comprising shall be understood as being open-ended and non-exclusive, *i.e*., "including but not limited to".

### BRIEF DESCRIPTION OF THE DRAWINGS

FIG. 1 is an XRPD pattern of the leucogen crystalline form A of the present disclosure.
FIG. 2 is an XRPD pattern of the leucogen crystalline form B of the present disclosure.
FIG. 3 is a TGA/DSC diagram for the leucogen crystalline form A.
FIG. 4 is a TGA/DSC diagram for the leucogen crystalline form B.

### DETAILED DESCRIPTION

The above-mentioned content of the present disclosure will be further illustrated in detail below with reference to the examples, but it shall not be understood to mean that the scope of the above subject matters of the present disclosure is limited only to the following examples. Any technologies implemented based on the above-mentioned content of the present disclosure are within the scope of the present disclosure.

### XRPD Test Method:

PANalytical X'Pert³ X-ray diffractometer was used for the XRPD test. An appropriate amount of samples were spread evenly and flatly on a single-crystal silicon sample pan. The XRPD test was carried out based on the parameters described below:

**Table 1 XRPD Test Parameters**

| | |
|---|---|
| Step Size [°2Th.]: 0.0263 | Scan Step Time [s]: 46.665 |
| K-Alpha1 [Å]: 1.54060 | K-Alpha2 [Å]: 1.54443 |
| Generator Settings: 40 mA, 45 kV | Scan Range [°2Th.]: 3-40 |

### TGA and DSC Test Methods:

TGA and DCS patterns were collected on TA Discovery TGA 5500 thermogravimetric analyzer and TA Discovery DSC 2500 differential scanning calorimeter, respectively. The test parameters were as follows:

**Table 2 TGA and DSC Test Parameters**

| | TGA | DSC |
|---|---|---|
| Sample Pan | Aluminum Pan (open) | Aluminum Pan (sealed) |
| Temperature range/°C | RT-~190~205 | 25-~190 |
| Scanning rate/°C/min | 10 | 10 |
| Protective gas | Nitrogen gas | Nitrogen gas |

Test on saturated solubility in water:
Chromatographic column: KromasiL C18 4.6*250 mm, 5 µm
Column temperature: 30°C flow rate: 1.0 mL/min sample size: 20 µL detection wavelength: 210 nm
Mobile phase: acetonitrile-water-glacial acetic acid (42:58:0.3)
Formulation of control solution: 10 mg of leucogen as a control product was precisely weighed, put in a 100-mL measuring flask, dissolved with the mobile phase, and diluted to the scale to afford 100 µg/mL of mother solution, and the mother solution was subjected to 10-fold, 100-fold, and 1000-fold serial dilutions to afford solutions at the concentrations of 10 µg/mL, 1 µg/mL, and 0.1 µg/mL, respectively.

Formulation of test solution: about 20 mg of 211118 batch (crystalline form A) and 211124 batch (crystalline form B) of the leucogen samples were weighed, each for 3 copies, and put in six 10-mL test tubes with stoppers respectively; 10 mL of water was added respectively; the test tubes were oscillated under the conditions of 37°C and 100 r/min for 1 h, 2 h, and 18 h respectively; the resultants were filtered through 0.45-µm microporous filter membranes, and the filtrates were taken as the test solutions.

The control solution and the test solutions were tested under the above chromatographic conditions, separately. The area of the control solution was linearly regressed with its concentration. The saturated solubility in water was obtained from the peak areas of the test solutions.

### Preparation Example: Preparation of leucogen compound

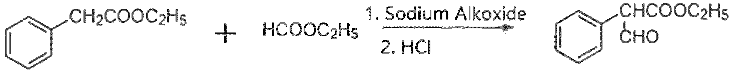

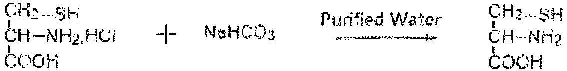

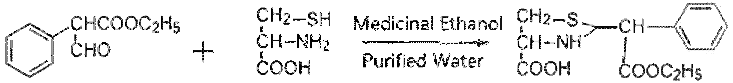

Ethyl phenylacetate (0.1 mol), 14.82 to 29.64 g of ethyl formate (0.2 to 0.4 mol), and sodium alkoxide (0.2 to 0.6 mol) were reacted. The reaction solution was controlled at 35±5°C and stirred for 5 to 6 h, and cooled to 0 to 10°C. 10% hydrochloric acid was slowly added to adjust the reaction solution to pH 1 to 3. The reaction solution was layered. The organic layer was washed with purified water, and distilled under reduced pressure to afford clear and transparent ethyl α-formylphenylacetate.

To an aqueous solution containing 0.1 mol of L-cysteine hydrochloride, an aqueous sodium bicarbonate solution was added for neutralization, and 0.1 to 0.2 mol of ethyl α-formylphenylacetate and an appropriate amount of medicinal ethanol were added to the reaction solution. The reaction system was heated to reflux, and reacted for 2 to 3 h. The reaction system was cooled to 10 to 20°C, added with purified water, and stirred for 5 to 6 h. The resultant was filtered. The filter cake was washed with an ethanol solution at 0 to 10°C, filtered, and dried to afford leucogen as a white solid.

¹H-NMR (600MHz, DMSO-*d₆*): δ 7.28 ~ 7.34 (m, 5H), 5.26 (d, *J* = 10.8 Hz, 1H), 4.05 (q, *J* = 7.2 Hz, 2H), 3.97 (m, 1H), 3.71 (d, *J =* 10.8 Hz, 1H), 3.12 (m, *J* = 8.1, 3.6 Hz, 1H), 2.72 (td, *J* = 7.2, 2.4 Hz, 1H), 1.12 (t, *J* = 7.2 Hz, 3H).

TOF-MS: calcd for C₁₄H₁₇NO₄S 296.0957 [M+H]⁺, found 296.0952 [M+H]⁺.

### Example 1

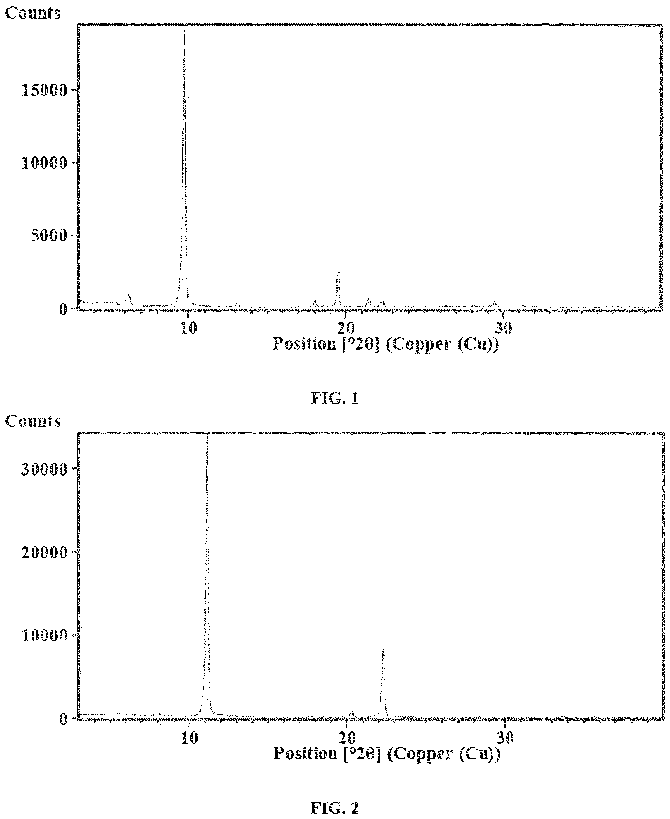

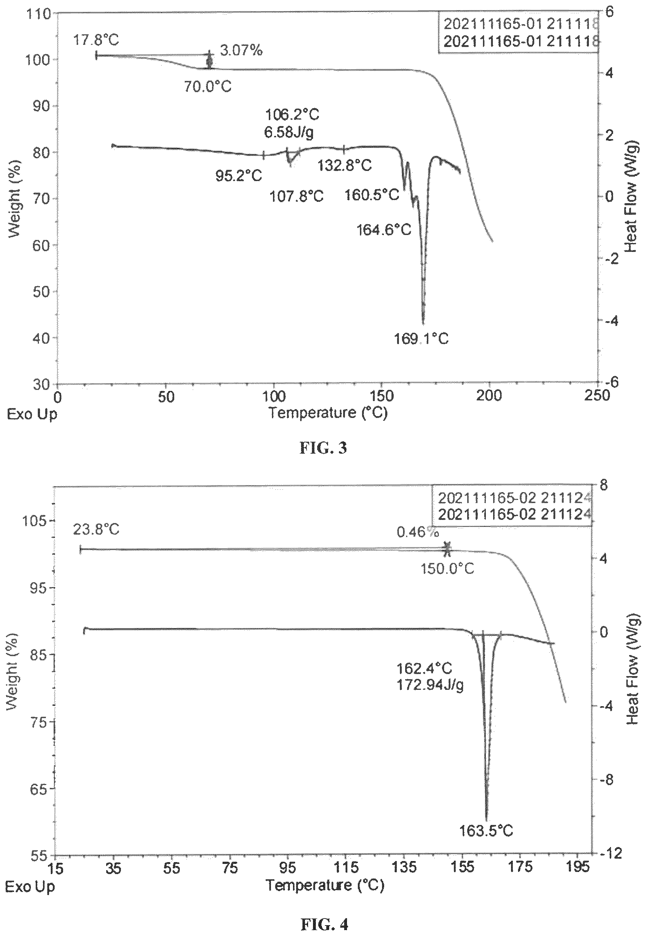

The preparation method for the crystalline form A of leucogen in the present embodiment was carried out according to the following steps:
2 g of the leucogen compound obtained in the Preparation Example was weighed, dissolved with stirring in 25 mL of *N,N*-dimethylformamide in a three-necked flask, and cooled in an ice-water bath after dissolution. When the internal temperature of the reaction flask was about 10°C, 25 mL of purified water was measured and added dropwise to the reaction flask, during which the temperature was controlled at 10°C±2°C. After completion of the dropwise addition, the reaction solution was stirred for 15 to 20 min while holding the temperature, and subjected to suction filtration. The filter cake was slurried and washed twice, and rinsed once with 20 mL of ice purified water respectively, and subjected to air-blowing drying at 70°C to afford 1 g of the crystalline form A of leucogen as powder. No methyl ester impurities (less than 0.01%) were detected in leucogen. The XRPD pattern was as shown in FIG. 1. The specific data on diffraction peaks were listed in Table 3. The TGA/DSC pattern was as shown in FIG. 3. The melting point was 161°C to 162°C.

**Table 3 Data on XRPD Diffraction Peaks**

| Pos. [°2θ] | Height [cts] | FWHM Left [°2θ] | d-spacing [Å] | Rel. Int. [%] |
|---|---|---|---|---|
| 4.8391 | 101.80 | 0.6140 | 18.26 | 0.53 |
| 6.2153 | 780.37 | 0.1023 | 14.22 | 4.08 |
| 9.7380 | 19110.18 | 0.1279 | 9.08 | 100.00 |
| 13.1302 | 315.39 | 0.1023 | 6.74 | 1.65 |
| 18.0722 | 482.79 | 0.1279 | 4.91 | 2.53 |
| 18.6252 | 127.25 | 0.1535 | 4.76 | 0.67 |
| 19.5155 | 2422.43 | 0.1279 | 4.55 | 12.68 |
| 21.4464 | 583.51 | 0.1279 | 4.14 | 3.05 |
| 22.3314 | 536.45 | 0.1535 | 3.98 | 2.81 |
| 23.6878 | 187.43 | 0.1279 | 3.76 | 0.98 |
| 25.0199 | 39.54 | 0.6140 | 3.56 | 0.21 |
| 26.3412 | 94.85 | 0.1535 | 3.38 | 0.50 |
| 27.0793 | 88.14 | 0.2047 | 3.29 | 0.46 |
| 28.0883 | 104.47 | 0.2047 | 3.18 | 0.55 |
| 29.3896 | 376.84 | 0.1023 | 3.04 | 1.97 |
| 31.2000 | 104.13 | 0.3582 | 2.87 | 0.54 |
| 37.9964 | 82.57 | 0.2047 | 2.37 | 0.43 |

### Example 2

The preparation method for the crystalline form B of leucogen in the present embodiment was carried out according to the following steps:
5 g of the leucogen compound obtained in the Preparation Example was weighed and put in a three-necked flask, to which 10 mL of dimethylformamide was added and subjected to magnetic stirring. The reaction system was heated to 80°C to 85°C in an oil bath until the solution was basically clear. Heating was terminated by removing the oil bath. The reaction system was cooled to 25°C to 30°C to precipitate a solid. The solid was subjected to suction filtration. The filter cake was slurried twice and rinsed once with 15 mL of tetrahydrofuran respectively, and subjected to air-blowing drying at 70°C to afford about 0.3 g of product, in which no methyl ester impurities (less than 0.01%) were detected. The XRPD pattern was as shown in FIG. 1. The specific data on diffraction peaks were listed in Table 4. The TGA/DSC pattern was as shown in FIG. 4. The melting point was 159°C to 160°C.

**Table 4 Data on XRPD Diffraction Peaks**

| Pos. [°2θ] | Height [cts] | FWHM Left [°2θ] | d-spacing [Å] | Rel. Int. [%] |
|---|---|---|---|---|
| 8.0245 | 511.97 | 0.1791 | 11.02 | 1.51 |
| 11.1151 | 34005.63 | 0.1279 | 7.96 | 100.00 |
| 17.6622 | 181.40 | 0.1023 | 5.02 | 0.53 |
| 20.3103 | 866.88 | 0.1535 | 4.37 | 2.55 |
| 22.2818 | 8150.84 | 0.1535 | 3.99 | 23.97 |
| 24.1149 | 79.79 | 0.3070 | 3.69 | 0.23 |
| 28.5456 | 273.98 | 0.1791 | 3.13 | 0.81 |
| 33.6668 | 139.14 | 0.2047 | 2.66 | 0.41 |
| 35.6712 | 86.11 | 0.1535 | 2.52 | 0.25 |

### Comparative Example 1

The preparation method for the crystalline form C of leucogen was carried out according to the following steps:
2 g of the leucogen compound obtained in the Preparation Example was weighed, dissolved with stirring in 25 mL of ethanol in a three-necked flask, and cooled in an ice water bath after dissolution. When the internal temperature of the reaction flask was about 10°C, 25 mL of purified water was measured and added dropwise to the reaction flask, during which the temperature was controlled at 10°C±2°C. After completion of the dropwise addition, the reaction solution was stirred for 15 to 20 min while holding the temperature, and subjected to suction filtration. The filter cake was slurried and washed twice and rinsed once with 20 mL of ice purified water respectively, and subjected to air-blowing drying at 70°C to afford 0.5 g of crystalline form C of leucogen as powder. The content of the methyl ester impurities in leucogen was 0.09%. The melting point was 157°C to 158°C.

### Example 3

The TGA/DCS data on the crystalline forms A and C of leucogen were listed in the table below:

**Table 5 TGA/DSC Test Results of Crystalline Forms A and C of Leucoen**

| Sample Group | DSC Endothermic Peak (peak temperature, °C) | TGA Weight Loss (%) |
|---|---|---|
| Crystalline form A | 132.8, 160.5, 164.6, 169.1 | 3.07 (100°C) |
| Crystalline form C | 95.2, 107.8 | 3.54 (70°C) |

The above table showed that the thermostability of the crystalline form A of leucogen was better than that of the crystalline form C.

### Example 4

The test data on water saturation solubility of the crystalline forms A and C of leucogen were listed in the table below:

**Table 6 Test Results of Water Saturation Solubility of Crystalline Forms A and C of Leucogen**

| Sample Group | Solubility in 1 h (µg/ml) | Solubility in 2 h (µg/ml) | Solubility in 18 h (µg/ml) |
|---|---|---|---|
| Crystalline form A | 115.33 | 137.28 | 241.02 |
| Crystalline form C | 112.64 | 133.16 | 227.89 |

The above table showed that the solubility property of the crystalline form A of leucogen was better than that of the crystalline form C.

### Example 5

The TGA/DCS data on the crystalline forms B and C of leucogen were listed in the table below:

**Table 7 TGA/DSC Test Results of Crystalline Forms B and C of Leucogen**

| Sample Group | DSC Endothermic Peak (peak temperature, °C) | TGA Weight Loss (%) |
|---|---|---|
| Crystalline form B | 162.4 | 0.46 (150°C) |
| Crystalline form C | 95.2, 107.8 | 3.54 (70°C) |

The above table showed that the thermostability of the crystalline form B of leucogen was better than that of the crystalline form C.

### Example 6

The test data on water saturation solubility of the crystalline forms B and C of leucogen were listed in the table below:

**Table 8 Test Results of Water Saturation Solubility of Crystalline Forms B and C of Leucogen**

| Sample Group | Solubility in 1 h (µg/ml) | Solubility in 2 h (µg/ml) | Solubility in 18 h (µg/ml) |
|---|---|---|---|
| Crystalline form B | 161.68 | 177.41 | 256.62 |
| Crystalline form C | 112.64 | 133.16 | 227.89 |

The above table showed that the solubility property of the crystalline form B of leucogen was better than that of the crystalline form C.

### Example 7

A total of 100 male Balb/C mice with the body weight ranging from 22 g to 24 g were randomly selected and randomly divided into the following five groups: the normal control group, the model control group, the leucogen crystalline form A group, the leucogen crystalline form B group, and the leucogen crystalline form C group, 20 mice in each group. In the leucogen crystalline forms A, B and C groups, the mice were intragastrically administered with 30 mg/kg once daily for a total of 28 days. In the normal control group and the model control group, the mice were administered with an equal volume of saline once daily for a total of 28 days. In the model control group and the leucogen groups, 25 mg/kg of cyclophosphamide was intraperitoneally injected in the mice at Day 8 to Day 14 respectively to establish mice leukopenia models. Blood samples were collected from the retro-orbital venous sinus of mice before modeling, after modeling, and at the end of drug administration (*i.e.*, Day 7, Day 15, and Day 29 after drug administration) for leukocyte counts, respectively. The experimental data were statistically processed with the SPSS 17.0 software package, and expressed as mean ± standard deviation (*X̅* ± *S*)*,* where P<0.05 indicated there was a statistical significance. The results were as shown in the table below.

**Table 9 Effects of Different Crystalline Forms of Leucogen on Total Number of Leukocytes in Mice (X̅ ± S)**

| Groups | Dose (mg/kg) | Leukocyte Level (10⁹ /L) | | |
|---|---|---|---|---|
| | | Before modeling | After modeling | End of treatment |
| Normal control group | - | 9.51±1.68 | 9.75±1.43* | 9.58±1.55* |
| Model control group | - | 9.85±1.54 | 3.23±0.69 | 4.78±1.45 |
| Leucogen crystalline form A group | 30 | 9.92±1.59 | 3.31±1.43 | 9.23±1.42*^{Δ} |
| Leucogen crystalline form B group | 30 | 9.63±0.99 | 3.46±1.24 | 8.98±1.13*^{Δ} |
| Leucogen crystalline form C group | 30 | 9.98±1.56 | 3.32±0.89 | 6.05±1.56* |

| | | | | |
|---|---|---|---|---|
| Note: *P<0.05 compared to the model control group; ΔP<0.05 compared to the leucogen C group. | | | | |

The above table showed that the peripheral blood leukocyte counts of the mice in each group after modeling were significantly decreased, all of which were less than 4.0×10⁹/L, indicating that the models were successfully established. After treatment, the leucocyte levels in all the leucogen crystalline forms A, B, and C groups could be significantly increased, and all of these groups exhibited significant differences as compared to the model control group (P<0.05). Among them, the leucogen crystalline forms A and B groups achieved a better effect, and had a significant difference as compared to the leucogen crystalline form C group (P<0.05).

The above examples are only the preferred technical solutions of the present disclosure, and shall not be regarded as limitations on the present disclosure. The scope of protection for the present disclosure shall be subject to the technical solutions disclosed in the claims, including the equivalent substitutions of the technical features in the technical solutions disclosed in the claims, that is, the equivalent substitutions and improvements within this scope are also within the scope of protection for the present disclosure.

## Claims

1. A crystalline form A of leucogen, wherein an X-ray powder diffraction pattern of the crystalline form A shows at least three diffraction peaks at 2θ angles of 6.215°±0.2°, 9.738°±0.2°, 13.130°±0.2°, 18.072°±0.2°, 19.516°±0.2°, 21.446°±0.2°, 22.331°±0.2°, 23.688°±0.2°, and 29.390°±0.2°.

2. The crystalline form A according to claim 1, wherein the X-ray powder diffraction pattern of the crystalline form A shows at least four diffraction peaks at 2θ angles of 6.215°±0.2°, 9.738°±0.2°, 13.130°±0.2°, 18.072°±0.2°, 19.516°±0.2°, 21.446°±0.2°, 22.331°±0.2°, 23.688°±0.2°, and 29.390°±0.2°.

3. The crystalline form A according to claim 2, wherein the X-ray powder diffraction pattern of the crystalline form A shows at least five diffraction peaks at 2θ angles of 4.839°±0.2°, 6.215°±0.2°, 9.738°±0.2°, 13.130°±0.2°, 18.072°±0.2°, 18.625°±0.2°, 19.516°±0.2°, 21.446°±0.2°, 22.331°±0.2°, 23.688°±0.2°, 25.020°±0.2°, 26.341°±0.2°, 27.079°±0.2°, 28.088°±0.2°, 29.390°±0.2°, 31.200°±0.2°, and 37.996°±0.2°.

4. The crystalline form A according to any one of claims 1 to 3, wherein the X-ray powder diffraction pattern of the crystalline form A shows at least seven diffraction peaks at 2Θ angles of 4.839°±0.2°, 6.215°±0.2°, 9.738°±0.2°, 13.130°±0.2°, 18.072°±0.2°, 18.625°±0.2°, 19.516°±0.2°, 21.446°±0.2°, 22.331°±0.2°, 23.688°±0.2°, 25.020°±0.2°, 26.341°±0.2°, 27.079°±0.2°, 28.088°±0.2°, 29.390°±0.2°, 31.200°±0.2°, and 37.996°±0.2°.

5. The crystalline form A according to claim 4, wherein a melting point is 161°C to 162°C.

6. A crystalline form B of leucogen, wherein an X-ray powder diffraction pattern of the crystalline form B shows at least three diffraction peaks at 2θ angles of 8.025°±0.2°, 11.115°±0.2°, 20.310°±0.2°, 22.282°±0.2°, 28.546°±0.2°, 33.667°±0.2°, and 35.671°±0.2°.

7. The crystalline form B according to claim 6, wherein the X-ray powder diffraction pattern of the crystalline form B shows at least four diffraction peaks at 2Θ angles of 8.025°±0.2°, 11.115°±0.2°, 20.310°±0.2°, 22.282°±0.2°, 28.546°±0.2°, 33.667°±0.2°, and 35.671°±0.2°.

8. The crystalline form B according to claim 7, wherein the X-ray powder diffraction pattern of the crystalline form B shows at least five diffraction peaks at 2Θ angles of 8.025°±0.2°, 11.115°±0.2°, 17.662°±0.2°, 20.310°±0.2°, 22.282°±0.2°, 24.115°±0.2°, 28.546°±0.2°, 33.667°±0.2°, and 35.671°±0.2°.

9. The crystalline form B according to any one of claims 6 to 8, wherein the X-ray powder diffraction pattern of the crystalline form B shows at least seven diffraction peaks at 2Θ angles of 8.025°±0.2°, 11.115°±0.2°, 17.662°±0.2°, 20.310°±0.2°, 22.282°±0.2°, 24.115°±0.2°, 28.546°±0.2°, 33.667°±0.2°, and 35.671°±0.2°.

10. The crystalline form B according to claim 9, wherein a melting point is 159°C to 160°C.

11. A method for preparing the crystalline form A of leucogen according to claim 1, wherein leucogen is dissolved with stirring in the presence of *N*,*N*-dimethylformamide, and subjected to cooling, water precipitation, suction filtration, and washing to afford a filter cake, and the filter cake is dried to afford the crystalline form A.

12. The preparation method according to claim 11, wherein temperatures of the dissolution and the stirring are both not higher than 15°C;
the volume ratio of the water to the *N*,*N*-dimethylformamide is 1:1; and
the drying is air-blowing drying at 60°C to 80°C.

13. A method for preparing the crystalline form B of leucogen according to claim 6, wherein leucogen is heated and dissolved in a *N*,*N*-dimethylformamide solvent under stirring conditions, and cooled to precipitate a solid, the solid is subjected to suction filtration, and a filter cake is washed and dried to afford the crystalline form B.

14. The preparation method according to claim 13, wherein temperatures of the dissolution and the stirring are both higher than 70°C;
the cooling is specifically cooling to 10°C to 30°C; and
the drying is air-blowing drying at 60°C to 80°C.

15. A pharmaceutical composition, comprising an effective amount of the crystalline form A of leucogen according to any one of claims 1 to 5 or the crystalline form B of leucogen according to any one of claims 6 to 10; preferably, further comprising a pharmaceutically acceptable excipient.

16. Use of the crystalline form A of leucogen according to any one of claims 1 to 5, or the crystalline form B of leucogen according to any one of claims 6 to 10, or the pharmaceutical composition according to claim 15 in the preparation of a medicament for preventing and/or treating a hematological system disease.

17. The use according to claim 16, wherein the hematological system disease is selected from leukopenia, thrombopenia, and aplastic anemia caused by various reasons.
